# EUROPEAN PATENT APPLICATION

(11) **EP 4 566 655 A1**
(43) Date of publication of application: **11.06.2025**
(21) Application number: 23213889.1
(22) Date of filing: 04.12.2023
(51) Int. Cl.: A61N 1/05, A61M 25/00

(54) **IMPLANTATION CATHETER FOR IMPLANTING AN ELECTRODE**

(71) Applicant: BIOTRONIK SE & Co. KG, 12359 Berlin (DE)
(72) Inventor: JOCHUM, Tobias, 13187 Berlin (DE); KOLBERG, Gernot, 12161 Berlin (DE)
(74) Representative: Biotronik Corporate Services SE

(57) **Abstract**

The invention relates to an implantation catheter (1) for implanting an electrode (12) comprising an electrode body (16) and a helical coil (13) configured to engage with tissue (15) at a location of interest, the helical coil (13) being arranged inside the electrode body (16) and being movable with respect to the electrode body (16) between a retracted position and an extended position, wherein the implantation catheter (1) comprises a catheter body (2) defining an electrode lumen (201) in its interior. In an aspect, the implantation catheter (1) further comprises a clamping device (10; 17) arranged in a distal portion (9) of the electrode lumen (201) and a clamping device operating arrangement (6; 18; 20) for transferring the clamping device (10; 17) between a non-clamping state and a clamping state, wherein the clamping device (10; 17) serves, in its clamping state, for limiting a rotational movement of an electrode body (16) of an electrode (12) arranged inside the electrode lumen (201).

## Description

The present invention relates to an implantation catheter according to the preamble of claim 1 and to an implantation catheter arrangement comprising such an implantation catheter according to the preamble of claim 14.

If an electrode with a retractable helix or another retractable fixation located inside an electrode body is guided through an implantation catheter to the intended tissue site of implantation and is then fixed in the tissue by turning and thus extending the helix, the electrode body can co-rotate with the helix and make the fixation of the electrode and the tissue difficult. Furthermore, a co-rotation of the electrode body may increase the risk of a later dislocation of the electrode.

According to a solution known from prior art, the electrode can be designed in a torsional stiff manner so that the electrode body will not co-rotate with the helix upon fixing the helix within tissue. However, it is constructively demanding to design an electrode to be torsion resistant and at the same time axially flexible. The stiffer the electrode, the higher is the risk of perforation of tissue.

For expanding the helix of an electrode, it is necessary that the electrode body applies a counter torque. If the electrode body is not constructed in a torsional stiff manner, this counter torque develops only by twisting the electrode body (due to co-rotation). Only if the counter torque is big enough to overcome the friction of the helix within the electrode body, the helix will expand due to the applied rotational force.

If the co-rotation of the electrode body is compensated by additional rotations of the helix, these additional rotations will be stored within the electrode body. After implantation, the stored additional rotations will be released and thus may cause the helix to unscrew out of the tissue. As a result, the electrode dislocates.

An additional disadvantage of the prior art solution is the poor contact pressure of the electrode onto the tissue. If the helix does not directly penetrate into the tissue, but makes unnecessary rotations within the blood due to lacking contact pressure, it will be expanded prior to be fully located within the tissue. Then, a gap between the tissue and the electrode body occurs through which electric stimulation energy, which is applied through the helix, is introduced into the blood and is thus lost for stimulation purposes. This increases the stimulation threshold of the electrode.

Yet another disadvantage of the prior art solution can be seen in that the electrode automatically pushes the implantation catheter back upon contacting the tissue if the electrode is pushed forward for applying a sufficiently high contact pressure. This may result in moving the implantation catheter back over the stiff area of the electrode head. The electrode may then laterally sidestep the contact pressure and finally bend down.

It is an object of the present invention to overcome these disadvantages of prior art and to provide a possibility of implanting an electrode in a more reliable manner, of reducing the risk of later dislocations of the electrode, of reducing the risk of damages of the electrode during implantation, and/or of reducing the risk of an increased stimulation threshold after having implanted the electrode.

This object is achieved with an implantation catheter for implanting an electrode having the features of claim 1. The electrode to be implanted by such an implantation catheter comprises an electrode body and a helical coil that is configured to engage with tissue at a location of interest. The helical coil (which can also be referred to as helix) is arranged inside the electrode body and is movable with respect to the electrode body between a retracted position and an extended position. This movement is accomplished by a rotation of the helical coil. Thus, upon rotation of the helical coil, it can be screwed into the tissue at the location of interest for implantation purposes or can be screwed out of this tissue for explantation purposes.

The implantation catheter further comprises a catheter body defining an electrode lumen in its interior. This electrode lumen serves for receiving the electrode to be implanted with the help of the implantation catheter.

According to an aspect of the present invention, the implantation catheter further comprises a clamping device arranged in a distal portion of the electrode lumen. Furthermore, the implantation catheter comprises a clamping device operating arrangement. This clamping device operating arrangement is designed and arranged for transferring the clamping device between a non-clamping state and a clamping state. Typically, the clamping device operating arrangement can transfer the clamping device from the non-clamping state to the clamping state and from the clamping state to the non-clamping state. However, in embodiments, only a transfer in one of these directions might be accomplished by the clamping device operating arrangement.

The clamping device is arranged and designed for allowing a basically unrestricted movement of an electrode inserted into the electrode lumen of the implantation catheter in the non-clamping state of the clamping device. The clamping device is furthermore arranged and designed for limiting a rotational movement of an electrode body of an electrode being present inside the electrode lumen in the clamping state of the clamping device. This limitation of a rotational movement is typically achieved by applying a high frictional force onto an outer surface of the electrode body. Then, a rotation of the electrode body will only be possible if a force is applied to the electrode body that is higher than the frictional force applied by the clamping device in its clamping state. Thus, the limitation of the rotational movement of the electrode body results, in an embodiment, in a restriction of any rotational movement due to a rotational force being smaller than the applied frictional force by the clamping device.

By selectively limiting a rotational movement of the electrode body, but still allowing a rotation of the helical coil, an easy relative movement between the coil and the electrode body can be accomplished that results in an extension or retraction of the helical coil with respect to the electrode body. No undesired torque will be applied to the electrode body when turning the helical coil into the tissue at the location of interest when the electrode body is fixed by the clamping device in its clamping state. This reduces the risk of any dislocations during and after the implantation.

Aspects of the presently claimed invention enable a user to insert an electrode in the usual manner into an implantation catheter and to freely move the electrode within the implantation catheter, but be able to fix the electrode during the time period of implanting the electrode into the tissue at the location of interest. Thus, the presently claimed implantation catheter enables a fixation of the electrode body during a rotational movement of the helical coil of the electrode for expanding the helical coil out of the electrode body and turning it into the tissue at the location of interest.

An electrode, which is fixed in this way in the tissue, experiences a much faster and more direct fixation than in case of prior art electrodes. The fixation mechanism responds more quickly to an applied force. Unnecessary rotations of the helical coil and the electrode body are no longer necessary. The electrode body does not store any undesired rotations that will be released at a later time point and may cause a dislocation of the electrode.

In addition, the contact pressure of the electrode onto the tissue is very reliable and high without encountering the risk that the electrode might bend down during applying the contact pressure. Due to the increased contact pressure, the electrode can also be implanted into harder tissue structures like those that can be found in the region of the His bundle of the human or animal heart.

The formation of a gap between tissue and electrode body is also avoided since the helical coil does not rotationally move within the blood of the patient, but directly turns into the tissue at the location of interest. This, in turn, results in a much lower risk of dislocation and in avoiding of an undesired high stimulation threshold.

The electrode can even be fixed within the tissue for a longer period during mapping (i.e., during electric scanning of cardiac or other tissue). The user only needs to move the implantation catheter for purposes of mapping and can rely on a stable position of the electrode within the implantation catheter. Injuries due to the mapping process are avoided since the interaction between the electrode and the implantation catheter is much better controlled than in case of prior art implantation catheters.

If it turns out that the electrode is too strongly fixated within the tissue prior to a desired change of position, it is possible to apply an additional force with the implantation catheter to the electrode tip to release the helical coil easier, in a better controlled and more atraumatic way than this is possible in case of prior art solutions.

In an embodiment, the distal portion of the electrode lumen, i.e., the portion in which the clamping device is arranged, extends over a range of from 0.5 mm to 5 cm, in particular of from 1 mm to 4.5 cm, in particular of from 2 mm to 4 cm, in particular of from 3 mm to 3.5 cm, in particular of from 4 mm to 3 cm, in particular of from 5 mm to 2.5 cm, in particular of from 6 mm to 2 cm, in particular of from 7 mm to 1.5 cm, in particular of from 8 mm to 1.1 cm, in particular of from 9 mm to 1.0 cm.

In an embodiment, the clamping device has a length, which is measured in a longitudinal extension direction of the implantation catheter, that lies in a range of from 0.5 mm to 5 cm, in particular of from 1 mm to 4.5 cm, in particular of from 2 mm to 4 cm, in particular of from 3 mm to 3.5 cm, in particular of from 4 mm to 3 cm, in particular of from 5 mm to 2.5 cm, in particular of from 6 mm to 2 cm, in particular of from 7 mm to 1.5 cm, in particular of from 8 mm to 1.1 cm, in particular of from 9 mm to 1.0 cm. If the clamping device is smaller than 0.5 mm, the clamping forces that can be applied by the clamping device in its clamping state will typically not be sufficient for limiting a rotational movement of an electrode body. On the other hand, a length of more than 5 cm is typically not necessary since the required forces can also be applied with a clamping device having a length of up to 5 cm.

In an embodiment, the distal end of the clamping device is flush with a distal end of the catheter body. In another embodiment, the distal end of the clamping device is not flush with the distal end of the catheter body, but rather is more proximally arranged than the distal end of the catheter body. Such an arrangement is connected with the effect that the clamping device is shielded by the catheter body against the surrounding tissue through which the implantation catheter is moved forward towards the location of interest. Thus, both the clamping device is protected against the tissue and the tissue is protected against the clamping device by the catheter body. Therefore, no severe injuries of the tissue needs to be feared due to any structures of the clamping device. In addition, and possibly even more important, no damages of the clamping device due to a contact with hard tissue structures need to be feared.

In an embodiment, an offset between the proximal end of the clamping device and the proximal end of the catheter body lies in a range of from 0.5 mm to 2 cm, in particular of from 0.6 mm to 1.5 cm, in particular of from 0.7 mm to 1.0 mm, in particular of from 0.8 mm to 0.9 mm.

In an embodiment, the clamping device comprises an expandable balloon. This balloon can be expanded by an appropriate inflation medium (also referred to as expansion medium), such as air, water, an aqueous solution like an aqueous sodium chloride solution, an oil, or a gel. An isotonic sodium chloride solution is a particularly appropriate inflation medium.

In an embodiment, the expandable balloon comprises or consists of a soft or flexible plastic material, such as a rubber-like material. In an embodiment, the plastic material is flexible.

In an embodiment, the expandable balloon is in fluid communication with the clamping device operating arrangement. Then, it is particularly easy to introduce an inflation medium with the clamping device operating arrangement into the expandable balloon or to remove it from the expandable balloon in order to bring the expandable balloon into its clamping state or its non-clamping state, respectively.

In an embodiment, the fluid communication is realized by a fluid canal which is at least partially arranged inside a wall of the catheter body. Such a fluid canal typically extends along a longitudinal extension direction of the implantation catheter between the expandable balloon and the clamping device operating arrangement.

In an embodiment, the clamping device operating arrangement comprises a fluid reservoir serving for storing the inflation medium to be applied into the expandable balloon or to be removed from the expandable balloon.

In an embodiment, the clamping device operating arrangement is arranged directly on an outside of the catheter body in a proximal portion of the catheter body. To give an example, the clamping device operating arrangement can be designed in form of a bladder that can be operated with the fingers of an operator by applying a slight pressure on it. The inflation medium being present in the bladder will then be pressed through the fluid canal into the expandable balloon and will serve for an expansion of the expandable balloon and thus for transferring the balloon from its non-clamping state into its clamping state.

In an embodiment, the clamping device operating arrangement comprises a lever or a button, the operation of which serves for letting an inflation medium flow from a fluid reservoir into the inflatable balloon. In an embodiment, the lever or button is arranged on the catheter body in a proximal region thereof.

In an embodiment, the clamping device operating arrangement is designed as a part being separate from the catheter body. But still in case of such a design as a separate part, a fluid communication between this separate part and the extendable balloon is established. To give an example, the clamping device operating arrangement can be designed in form of a syringe which is connected with a tube with the expandable balloon or with a fluid canal which, in turn, is fluidly connected with the expandable balloon. By moving the cylinder of the syringe, an inflation medium can then be transferred from the syringe into the expandable balloon and vice versa in order to transfer the expandable balloon between its clamping (expanded) state and its non-clamping (retracted) state.

In an embodiment, the clamping device comprises an elastic ring that is typically arranged around the whole inner circumference of the catheter body in the distal portion of the catheter body. Such an elastic ring is typically not inflatable or expandable, but can alter its shape and can thus be present in a clamping state or in a non-clamping state. To give an example, by applying a force onto the elastic ring, it will bend to an inside of the electrode lumen, since there is no space for an expansion towards the inner wall of the catheter body itself. By bending towards an inside of the electrode lumen, the available diameter of the catheter lumen will be reduced and the clamping force will be applied to a catheter body of the catheter being present in the catheter lumen.

In an embodiment, the clamping device operating arrangement comprises a tube that is arranged inside the electrode lumen. This tube is operatively coupled to the clamping device. To give an example, it can simply abut with its distal end portion against the proximal end portion of the elastic ring of the clamping device. A movement of the tube in the longitudinal extension direction of the implantation catheter will then serve for transferring the clamping device between the clamping state and the non-clamping state due to exerting a different pressure onto the clamping device. E.g., by distally moving the tube in the longitudinal extension direction of the implantation catheter, the elastic ring is compressed and bends towards the interior of the electrode lumen. If an electrode is placed within the electrode lumen, the elastic ring will then abut against an outside of the electrode body and will thus apply a frictional force onto the electrode body. By proximally moving the tube in the longitudinal extension direction of the implantation catheter, the elastic ring is released again and moves back to the inner wall of the electrode body. The frictional force against an electrode body of an electrode being present within the electrode lumen will then be released again.

In an embodiment, the clamping device operating arrangement comprises a traction cable that is operatively coupled to the clamping device. A movement of the traction cable in a longitudinal extension direction of the implantation catheter will then serve for transferring the clamping device between the clamping state and the non-clamping state. For this purpose, the traction cable is, in an embodiment, connected to a movable stop ring positioned distally of the elastic ring. The elastic ring abuts with its distal and against the proximal end of the stop ring. If the traction cable is pulled, the movable stop ring will be moved in a proximal direction. It will then serve for a compression of the elastic ring and for a bending of the elastic ring to an inside of the electrode lumen. Thus, this movement transfers the elastic ring from its non-clamping state into its clamping state. If the pulling force onto the traction cable is released again, the movable stop ring will move back to its original position, thus releasing the force being applied onto the elastic ring. As a result, the elastic ring will be transferred into its non-clamping state and will release a frictional force applied onto an outside of the electrode body of an electrode being present within the electrode lumen of the implantation catheter.

In an embodiment, the traction cable is connected to a movable stop ring being arranged proximally to a proximal end of the elastic ring. This movable stop ring is arranged under a pretension and abuts against the elastic ring. If the traction cable is pulled in a proximal direction along the longitudinal extension direction of the implantation catheter, the movable stop ring is moved against the pretensioning force and thus releases a pretension that has been previously applied onto the elastic ring. Then, the elastic ring is transferred from its clamping state into its non-clamping state. Thus, in this arrangement, pulling the traction cable will release a frictional force that is applied by the elastic ring onto an outside of an electrode body of an electrode being present in the electrode lumen of the implantation catheter as long as the traction cable is not pulled.

In an embodiment, the implantation catheter serves for implanting a cardiac electrode, in particular a cardiac electrode for sensing and/or stimulating the His bundle of a human or animal heart.

In an aspect, the present invention relates to an implantation catheter arrangement that comprises an implantation catheter according to the preceding explanations and an electrode arranged inside the electrode lumen of this implantation catheter. The electrode comprises an electrode body and a helical coil configured to engage with tissue at a location of interest. The helical coil is arranged inside the electrode body and can be moved with respect to the electrode body between a retracted position and an extended position. The clamping device of the implantation catheter is able to apply a frictional force between the catheter body and the electrode body so as to limit rotational (and axial) movements of the electrode body if the clamping device is in its clamping state.

In an embodiment, the helical coil protrudes in the retracted position from the electrode body by a protruding length such that the helical coil can be brought into engagement with tissue even in its retracted position. After having established such an engagement with the tissue, it is particularly easy to apply a rotational force onto the helical coil and to turn the helically coil into the tissue, while clamping the electrode body with the clamping device of the implantation catheter and avoiding the introduction of an undesired torque into the electrode body.

In an aspect, the present invention relates to a medical method for implanting an electrode into the body of a patient in need thereof. This method comprises the steps explained in the following.

First, the electrode to be implanted is guided into an electrode lumen of an implantation catheter according to the preceding explanations. The electrode comprises an electrode body and a helical coil configured to engage with tissue at a location of interest. The helical coil is arranged inside the electrode body and can be moved with respect to the electrode body between a retracted position and an extended position.

The implantation catheter and the electrode are then guided towards a location of interest, i.e., an intended implantation site.

At least by now, the clamping device of the implantation catheter is transferred from its non-clamping state into its clamping state in order to apply a frictional force onto an outside of the electrode body. Upon doing so, rotational (and axial) movements of the electrode body are strongly limited. As long as typical rotational forces are applied onto the helical coil, no rotational movement of the electrode body will occur due to the frictional force.

The helical coil is then turned into the tissue at the intended implantation site by rotating the helically coil inside the electrode body.

Once the helical coil has reached its intended final position, the clamping device is transferred from its clamping state into its non-clamping state so that the previously applied frictional force is released. Then, the implantation catheter can be proximally moved and retracted from the implanted electrode that remains in the body of the patient.

In an embodiment, the electrode is a cardiac electrode, in particular a His bundle electrode.

All embodiments of the implantation catheter can be combined in any desired way and can be transferred either individually or in any arbitrary combination to the implantation catheter arrangement and to the described method. All embodiments of the implantation catheter arrangement can be combined in any desired way and can be transferred either individually or in any arbitrary combination to the implantation catheter and to the described method. Finally, all embodiments of the method can be combined in any desired way and can be transferred either individually or in any arbitrary combination to the implantation catheter and to the implantation catheter arrangement.

Further details of aspects of the present invention will be explained in the following making reference to exemplary embodiments and accompanying Figures. In the Figures:
- Fig. 1: shows a schematic view of an implantation catheter with a syringe connected to the catheter via a tube;
- Fig. 2A: shows a schematic longitudinal section through a first embodiment of an implantation catheter in a first state;
- Fig. 2B: shows a schematic longitudinal section through the implantation catheter of Figure 2A in a second state;
- Fig. 3A: shows a schematic longitudinal section through the implantation catheter of Figures 2A and 2B with inserted electrode in a first state of the electrode;
- Fig. 3B: shows a schematic longitudinal section through the implantation catheter of Figures 2A and 2B with inserted electrode in a second state of the electrode;
- Fig. 4A: shows a schematic longitudinal section through a second embodiment of an implantation catheter in a first state;
- Fig. 4B: shows a schematic longitudinal section through the implantation catheter of Figure 4A in a second state; and
- Fig. 5: shows a longitudinal section through an embodiment of an implantation catheter with a clamping device operating arrangement.

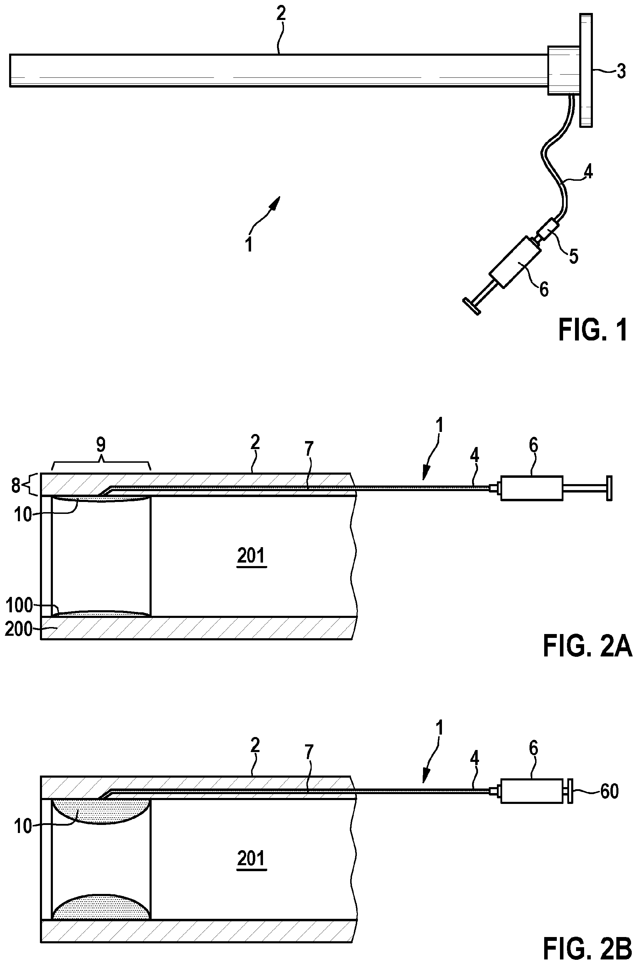

Figure 1 shows an embodiment of an implantation catheter 1 comprising a catheter shaft 2 that is to be inserted into the blood system of a patient. The catheter shaft 2 serves as catheter body. The implantation catheter 1 comprises a handle 3 at its proximal end, with the help of which the catheter shaft 2 of the implantation catheter 1 can be moved through the blood system of the patient. Furthermore, the handle 3 can comprise manipulating elements by which the catheter shaft 2 can be slit or split after a successful implantation.

At its proximal end, the implantation catheter 1 is connected with a tube 4 comprising a Luer lock connector 5. A syringe 6 is connected to the Luer lock adapter 5. An inflation medium being present in the syringe 6 can be pushed through the tube 4 into a canal (not shown) of the implantation catheter 1. Such a mode of operation is shown in more detail in Figures 2A and 2B.

Figure 2A shows a longitudinal section through the catheter shaft 2 of the implantation catheter 1 of Figure 1. In this and all following Figures, similar elements will be denoted with the same numeral reference.

In the depiction of Figure 2A, it can be seen that the syringe 6 is connected via the tube 4 with a canal 7 that is arranged within a sidewall 8 of the catheter shaft 2 of the implantation catheter 1.

In a distal end section 9 of the catheter shaft 2 of the implantation catheter 1, an inflatable balloon 10 is arranged that is in fluid communication with the canal 7. This an inflatable balloon 10 serves as clamping device. A distal end 100 of the inflatable balloon 10 is not fully flush with a distal end 200 of the catheter shaft 2 of the implantation catheter 1. Rather, there remains a small distance between the distal and 100 of the inflatable balloon 10 and the distal end 200 of an electrode lumen 201 of the catheter shaft 2 defined by the sidewall 8 of the catheter shaft 2.

Upon pressing onto a piston 60 of the syringe 6, an inflation medium being present within the syringe 6 is pressed through the tube 4 and the canal 7 into the inflatable balloon 10 and serves for an inflation of this inflatable balloon 10. Consequently, the inflated inflatable balloon 10 exhibits a clamping force onto an electrode if present inside the electrode lumen 201.

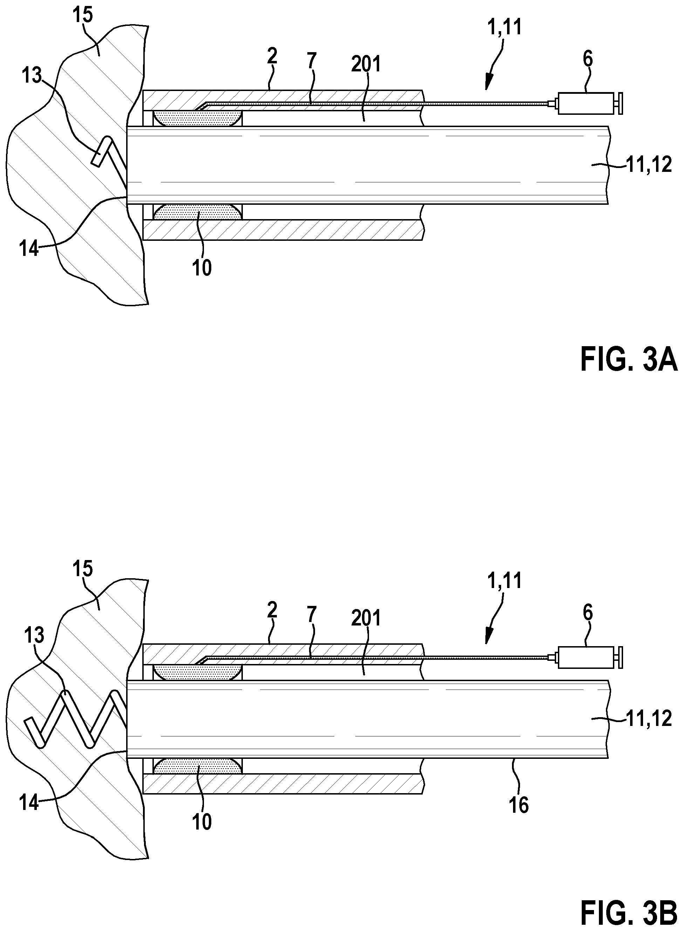

Figure 3A shows an implantation catheter arrangement 11 comprising an implantation catheter 1 and an electrode 12 inserted into the electrode lumen 201 inside the catheter shaft 2 of the implantation catheter 1. The implantation catheter 1 is the same implantation catheter 1 shown in Figures 2A and 2B and discussed above. In a distal portion of the electrode 12, the electrode 12 is clamped by the inflatable balloon 10 in its inflated state. A helical coil 13 of the electrode 12 slightly protrudes a distal end 14 of the electrode 12. In this state, the catheter tip can be moved over tissue 15 in order to look for appropriate locations for implanting the electrode 12. Typically, the final site of implantation is chosen according to the electric conductivity of the tissue 15 at the site of implantation. This process of looking for an appropriate site of implantation is also referred to as mapping.

Due to the clamping by the inflated inflatable balloon 10, the electrode 12 is arrested inside the electrode lumen 201. A physician handling the implantation catheter arrangement 11 does not need to care whether the electrode 12 is sufficiently advanced within the implantation catheter 1 since the position of the electrode 12 within the implantation catheter 1 is fixed.

Once an appropriate site of implantation is found, the helical coil 13 can be further advanced into the tissue 15 by rotating it within an electrode body 16 of the electrode 12. Due to the clamping force applied by the inflatable balloon 10 in its inflated state, the electrode body 16 remains stable in place even though a torque is applied due to the rotation of the helical coil 13. The anchoring of the electrode 12 with the help of the helical coil 13 within the tissue 15 can be achieved in a very stable way since the electrode 12 can be tightly pressed against the tissue 15 due to its clamping by the inflatable balloon 10 in its inflated state. As a result, the electrode coil 13 can also be anchored within hard tissue structures.

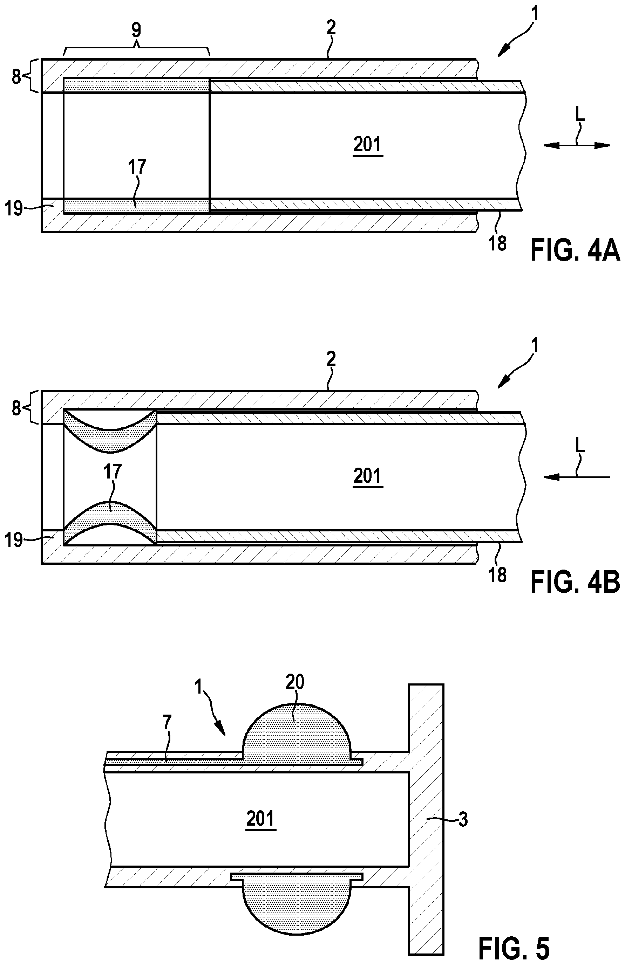

Figure 4A shows another embodiment of an implantation catheter 1. In this embodiment, the implantation catheter 1 does not comprise an inflatable balloon, but rather an elastic ring 17 that is arranged in a distal end portion 9 of the electrode lumen 201 defined by the wall 8 of the catheter shaft 2. The elastic ring 17 is arranged circumferentially inside the electrode lumen 201 and abuts against an inner surface of the wall 8 of the catheter shaft 2.

A movable tube 18 is arranged inside the catheter shaft 2, wherein an interior of the movable tube 18 defines a section of the electrode lumen 201 in this embodiment. The movable tube 18 can be moved along a longitudinal direction of extension L, as indicated by an arrow in Figure 4A.

The elastic ring 17 is supported by a stop 19 that is arranged in the very distal end portion of the catheter shaft 2 (distal to the distal end portion 9 of the electrode lumen 201 in which the elastic ring 17 is positioned). Due to this stop 19, any force applied by the movable tube 18 will be directly introduced into the elastic ring 17.

Upon pushing the movable tube 18 distally in the longitudinal extension direction of the implantation catheter 1, it exerts a force onto the elastic ring 17, thus deforming the elastic ring 17. This deformation results in exerting a clamping force onto an electrode being present inside the electrode lumen 201.

Thus, the embodiment shown in Figures 4A and 4B does not require a syringe for providing an inflation medium. Rather, it allows the application of a clamping force by the elastic ring 17 simply by advancing the movable tube 18 in a distal direction along the longitudinal extension direction L of the implantation catheter 1.

In a variant of the embodiment shown in Figures 4A and 4B, the stop 19 is made movable by a traction cable or a similar operating element so that a force can be applied onto the elastic ring 17 by moving the stop 19 proximally along the longitudinal extension direction L.

Figure 5 shows the proximal portion of another embodiment of an implantation catheter 1. Here, a small bladder 20 is arranged next to the handle 3. The bladder 20 serves as a reservoir for an inflation medium. By exerting a pressure onto the bladder 20, the inflation medium will be pressed through the canal 7 towards the inflatable balloon 10 (not shown in Figure 5; cf. Figures 2A to 3B). The bladder 20 can be arranged - as shown in Figure 5 - circumferentially around the whole circumference of the catheter shaft 2. In another embodiment, it can only be arranged at a portion of the circumference of the catheter shaft 2.

This arrangement of the bladder 20 makes it possible that a physician can control the clamping force applied by the inflatable balloon 10 in a very differentiated way. The physician will also be provided with haptic feedback if the pressure conditions at the inflatable balloon 10 change. This is due to the fact that the canal 7 provides a direct fluid connection between the inflatable balloon 10 and the bladder 20, enabling such a direct haptic feedback.

## Claims

1. Implantation catheter (1) for implanting an electrode (12) comprising an electrode body (16) and a helical coil (13) configured to engage with tissue (15) at a location of interest, the helical coil (13) being arranged inside the electrode body (16) and being movable with respect to the electrode body (16) between a retracted position and an extended position, wherein the implantation catheter (1) comprises a catheter body (2) defining an electrode lumen (201) in its interior,
**characterized**
**in that** the implantation catheter (1) further comprises a clamping device (10; 17) arranged in a distal portion (9) of the electrode lumen (201) and a clamping device operating arrangement (6; 18; 20) for transferring the clamping device (10; 17) between a non-clamping state and a clamping state, wherein the clamping device (10; 17) serves, in its clamping state, for limiting a rotational movement of an electrode body (16) of an electrode (12) arranged inside the electrode lumen (201).

2. Implantation catheter according to claim 1, **characterized in that** the distal portion (9) of the electrode lumen (201), in which the clamping device (10; 17) is arranged, extends over a range of from 0.5 mm to 5 cm.

3. Implantation catheter according to claim 1 or 2, **characterized in that** the clamping device (10; 17) has a length, measured in a longitudinal extension direction (L) of the implantation catheter (1), lying in a range of from 0.5 mm to 5 cm.

4. Implantation catheter according to any of the preceding claims, **characterized in that** a distal end (100) of the clamping (10; 17) device is more proximally arranged than a distal end (200) of the catheter body (2).

5. Implantation catheter according to any of the preceding claims, **characterized in that** the clamping device (10; 17) comprises an expandable balloon (10).

6. Implantation catheter according to claim 5, **characterized in that** the expandable balloon (10) is in fluid communication with the clamping device operating arrangement (6; 20).

7. Implantation catheter according to claim 6, **characterized in that** the fluid communication is realized by a fluid canal (7) at least partially arranged inside a wall (8) of the catheter body (2).

8. Implantation catheter according to any of claims 5 to 7, **characterized in that** the clamping device operating arrangement (6; 20) comprises a fluid reservoir.

9. Implantation catheter according to any of claims 5 to 8, **characterized in that** the clamping device operating arrangement (6; 20) is arranged directly on an outside of the catheter body (2) in a proximal portion of the catheter body (2).

10. Implantation catheter according to any of claims 5 to 8, **characterized in that** the clamping device operating arrangement (6; 20) is designed as a component (6) being separate from the catheter body (2) but being in fluid communication with the expandable balloon (10).

11. Implantation catheter according to any of claims 1 to 4, **characterized in that** the clamping device (10; 17) comprises an elastic ring (17).

12. Implantation catheter according to claim 11, **characterized in that** the clamping device operating arrangement (6; 18; 20) comprises a tube (18) arranged inside the electrode lumen (201) and being operatively coupled to the clamping device (17), wherein a movement of the tube (18) in a longitudinal extension direction (L) of the implantation catheter (1) serves for transferring the clamping device (17) between the clamping state and the non-clamping state.

13. Implantation catheter according to claim 11, **characterized in that** the clamping device operating arrangement (6; 18; 20) comprises a traction cable operatively coupled to the clamping device (10; 17), wherein a movement of the traction cable in a longitudinal extension direction (L) of the implantation catheter (1) serves for transferring the clamping device (10; 17) between the clamping state and the non-clamping state.

14. Implantation catheter arrangement (11) comprising an implantation catheter (1) according to any of the preceding claims and an electrode (12) arranged inside the electrode lumen (201), wherein the electrode (12) comprises an electrode body (16) and a helical coil (13) configured to engage with tissue (15) at a location of interest, the helical coil (13) being arranged inside the electrode body (16) and being movable with respect to the electrode body (16) between a retracted position and an extended position.

15. Implantation catheter arrangement according to claim 14, **characterized in that** helical coil (13) in the retracted position protrudes from the electrode body (16) by a protruding length such that the helical coil (13) is functional to be brought into engagement with tissue (15) in the retracted position.
